# EUROPÄISCHE PATENTANMELDUNG

(11) **EP 1 867 283 A1**
(43) Veröffentlichungstag der Anmeldung: **19.12.2007**
(21) Anmeldenummer: 07107407.4
(22) Anmeldetag: 03.05.2007
(51) Int. Cl.: A61B 6/00, A61B 6/04

(54) **Röntgenvorrichtung mit keimhemmender Schicht**

(30) Priorität: 13.06.2006 DE 102006027416
(71) Anmelder: SIEMENS AKTIENGESELLSCHAFT, 80333 München (DE)
(72) Erfinder: Hörnig, Mathias, 91052, Erlangen (DE)

(57) **Zusammenfassung**

Die Erfindung betrifft eine Röntgenvorrichtung (10) zur Bildaufnahme eines zu untersuchenden Teils (B) eines Untersuchungsobjekts, mit einem Röntgenstrahler (21) und einem Röntgendetektor (22), zwischen welchen sich ein Strahlbereich (X) erstreckt, in dem der zu untersuchende Teil (B) des Untersuchungsobjekts positionierbar ist, wobei ein Objekttisch (32) zur Positionierung des zu untersuchenden Teils (B) des Untersuchungsobjekts im Strahlbereich (X) und eine relativ zum Objekttisch (32) verstellbare Kompressionseinrichtung (30) mit einer Kompressionsplatte (31) vorgesehen sind, wobei der zu untersuchende Teil (B) des Untersuchungsobjekts mittels des Objekttisches (32) und der Kompressionsplatte (31) komprimierbar ist. Indem wenigstens ein Teil (41, 51) der Kompressionsplatte (31) und/oder wenigstens ein Teil (42, 52) des Objekttisches (32) derart ausgebildet ist, dass eine Hemmung einer Keimentwicklung bewirkt wird, kann eine Röntgenvorrichtung bereitgestellt werden, welche die Hygienezustände bei Röntgenuntersuchungen verbessert ohne die Wirtschaftlichkeit des Einsatzes der Röntgeneinrichtung zu verringern.

## Beschreibung

Die Erfindung betrifft eine Röntgenvorrichtung zur Bildaufnahme eines zu untersuchenden Teils eines Untersuchungsobjekts, mit einem Röntgenstrahler und einem Röntgendetektor, zwischen welchen sich ein Strahlbereich erstreckt, in dem der zu untersuchende Teil des Untersuchungsobjekts positionierbar ist, wobei ein Objekttisch zur Positionierung des zu untersuchenden Teils des Untersuchungsobjekts im Strahlbereich und eine relativ zum Objekttisch verstellbare Kompressionseinrichtung mit einer Kompressionsplatte vorgesehen sind, wobei der zu untersuchende Teil des Untersuchungsobjekts mittels des Objekttisches und der Kompressionsplatte komprimierbar ist.

Röntgenvorrichtungen bilden trotz fortschreitender Entwicklung und neuer Möglichkeiten im Bereich strahlungsfreier medizinischer Diagnostik eine wesentliche Stütze der Medizintechnik. Röntgenvorrichtungen sind dabei in einer großen Anzahl von medizinischen Einsatzgebieten anzutreffen. Die Einsatzgebiete reichen von der Röntgendiagnostik, beispielsweise von der Aufklärung von Knochenfrakturen, Tumoren, Zysten, Verkalkungen, Lufteinschlüssen oder auch Vorsorgeuntersuchungen, bis zu fluoroskopischen Untersuchungen, etwa bei Angiographien, Überwachung von medizinischen Interventionen oder Lokalisierung medizinischer Instrumente.

Für die Brustkrebsvorsorge sowie für den Nachweis von Kalzifizierungen in der weiblichen Brust werden häufig Mammographievorrichtungen genutzt. Dabei wird die Brust auf einem Objekttisch platziert und insbesondere mittels einer relativ zum Objekttisch vertikal verstellbaren Kompressionsplatte komprimiert. Die Kompression der Brust dient zur Reduktion von Röntgenstreustrahlen sowie zur Herstellung einer näherungsweise konstanten Dicke der zu untersuchenden Brust. Weiter wird durch die Kompression die Brust vom Thorax beabstandet, wodurch eine oberflächennahe Untersuchung der Brust ermöglicht wird.

Die Kompression eines zu untersuchenden Teils eines Untersuchungsobjekts kann bspw. auch bei Punktionen oder Biopsien eines Untersuchungsobjekts verwendet werden. Die Punktion oder Biopsie kann dabei ggf. mittels röntgenbasierten Bildaufnahmen, wie sie etwa durch ein C-Bogen-Röntgengerät, durch ein Computertomographiegerät oder ein Magnetresonanztomographiegerät geleistet werden können, kontrolliert werden.

Es sind verschiedene Ausführungen von Kompressionsplatten bekannt, die in der Regel aus einem für Röntgenstrahlen transparenten oder nahezu transparenten Material gefertigt sind. Je nach Anwendungsgebiet und Brustgröße sind unterschiedliche Kompressionsplatten vorgesehen. Daher muss der Anwender des Mammographiegeräts in der Regel einige verschiedene Typen von Kompressionsplatten vorrätig halten.

Dennoch wird im klinischen Betrieb aus verschiedenen Gründen kein häufiger Wechsel der Kompressionsplatten durchgeführt, insbesondere dann nicht, wenn ein hoher Durchsatz an Patienten gewünscht ist - wie etwa bei Vorsorgeuntersuchungen bzw. bei gleichen medizinischen Fragestellungen - oder wenn die Patienten ähnlich große Brüste aufweisen.

Bei der Untersuchung kommen die Patienten somit mit einer Kompressionsplatte in Kontakt, die zuvor bei einer Untersuchung eines anderen Patienten genutzt und dabei ggf. verunreinigt bzw. mit Keimen kontaminiert wurde. Auch der Austausch einer Kompressionsplatte führt in der Regel zu einem Kontakt des medizinischen Personals mit der Kompressionsplatte und damit ggf. zu Verschmutzung und Keimeintrag auf die Kompressionsplatte, wobei in der Regel nach dem vorgenommenen Austausch keine Zwischenreinigung der Kompressionsplatte vorgesehen ist.

Rückstände auf der Kompressionsplatte und/oder dem Objekttisch in Form von Schmutz oder Keimen, wie etwa Bakterien, die von der Berührung der Kompressionsplatten durch andere Patienten oder klinischem Personal stammen, sind aus Hygienegründen nicht erwünscht. Eine regelmäßige Reinigung der Kompressionsplatte und/oder des Objekttisches ist jedoch unwirtschaftlich, da zeitaufwendig und erschwert einen klinischen Arbeitsablauf.

Der Erfindung liegt die Aufgabe zugrunde, eine gattungsgemäße Röntgenvorrichtung der eingangs genannten Art bereitzustellen, welche die Hygienezustände bei Röntgenuntersuchungen verbessert, ohne die Wirtschaftlichkeit des Einsatzes der Röntgeneinrichtung zu verringern.

Die Aufgabe wird bei einer gattungsgemäßen Röntgenvorrichtung der eingangs genannten Art dadurch gelöst, dass wenigstens ein Teil der Kompressionsplatte und/oder wenigstens ein Teil des Objekttisches derart ausgebildet ist, dass eine Hemmung einer Keimentwicklung bewirkt wird. Unter Hemmung einer Keimentwicklung kann dabei sowohl das Abtöten von Keimen bzw. einer Keimpopulation - wie etwa eine Bakterienpopulation - verstanden werden, als auch eine Verhinderung oder Reduzierung des Wachstums einer Keimpopulation. Sowohl durch das Abtöten von Keimen als auch durch die Reduktion der natürlichen Wachstumsrate der Keimpopulation oder verschiedener Keimpopulationen kann die Hygiene verbessert werden.

Vorzugsweise sind sowohl Objekttisch als auch Kompressionsplatte, insbesondere jeweils jener Teil, der mit dem zu untersuchenden Teil des Untersuchungsobjekts - bspw. einer weiblichen Brust - während der Untersuchung in physischem Kontakt steht, derart ausgebildet, dass eine Hemmung einer Keimentwicklung bewirkt wird. Die erfindungsgemäße Ausbildung des Objekttisches und/oder der Kompressionsplatte erlaubt es, das Wachstum von verschiedensten Mikroorganismen empfindlich zu stören, wodurch die Übertragung und Ausbreitung von Infektionen zwischen verschiedenen Untersuchungsobjekten verringert wird.

Eine Reinigung der Kompressionsplatte und/oder des Objekttisches kann mit der erfindungsgemäßen Lösung entfallen, d.h. der Durchsatz bspw. von Patienten kann bei verbesserten hygienischen Bedingungen auf hohem Niveau gehalten werden.

Bei der erfindungsgemäßen Ausbildung von Objekttisch und/oder Kompressionsplatte ist es in der Regel erforderlich, die Verträglichkeit der zur Hemmung einer Keimentwicklung verwendeten Materialen mit dem Patienten zu berücksichtigen. Es können also in der Regel Materialien zum Einsatz kommen, welche zum einen eine Keimentwicklung hemmende Wirkung bereitstellen und zum anderen für einen Patienten keine belastenden Nebenwirkungen besitzen.

In einer vorteilhaften Ausgestaltung der Erfindung weist wenigstens ein Teil der Kompressionsplatte und/oder wenigstens ein Teil des Objekttisches eine Keimentwicklung hemmende Beschichtung auf. Die Beschichtung kann mit beliebigen Verfahren vorgenommen werden, bspw. durch Aufdampfen der Keimentwicklung hemmenden Beschichtung oder durch eine Fällungsreaktion, bei der die Schicht aus einer Lösung abgeschieden wird. Die verwendbaren Verfahren zum Aufbringen einer die Keimentwicklung hemmenden Beschichtung wenigstens auf einen Teil der Kompressionsplatte und/oder wenigstens auf einen Teil des Objekttisches hängen stark von dem verwendeten Beschichtungsmaterial ab.

Bei der Beschichtung kann eine Maskierung der Kompressionsplatte und/oder des Objekttisches vorgenommen werden, so dass nur jener Teil der Kompressionsplatte und/oder des Objekttisches beschichtet wird, welcher später auch in Kontakt mit dem zu untersuchenden Teil des Untersuchungsobjekts steht. Durch die Beschichtung nur eines Teils des Objekttisches oder der Kompressionsplatte mit einem die Keimentwicklung hemmenden Material können Kosten eingespart werden, da eine geringere Menge von Keimentwicklung hemmendem Material benötigt wird.

In einer vorteilhaften Ausführungsform der Erfindung ist die Beschichtung als eine Silberschicht ausgebildet. Dabei ist zu berücksichtigen, dass die auf die Kompressionsplatte und/oder auf den Objekttisch aufgebrachte Silberschicht von einer Dicke ist, welche die während der Untersuchung durch die Silberschicht hindurch tretenden Röntgenstrahlen nur in vernachlässigbarer Weise absorbiert und/oder streut. Mit Silber steht ein chemisches Element zur Verfügung, mit welchem auf einfache und gut handhabbare Weise eine Keimentwicklung hemmende Wirkung erzielt werden kann. Dabei kann die Silberbeschichtung nur in jenem Bereich der Kompressionsplatte und/oder des Objekttisches vorgesehen werden, der mit dem zu untersuchenden Teil des Untersuchungsobjekts in direktem physischen Kontakt steht.

Das Silber kann auch in Form von Nanopartikeln auf den Objekttisch und/oder auf die Kompressionsplatte aufgebracht werden, um die wirksame Oberfläche, welche zur Hemmung der Keimentwicklung vorgesehen ist, zu erhöhen.

In einer weiteren vorteilhaften Ausführungsform der Erfindung ist die Beschichtung als eine Vielzahl von jeweils mit Silber überzogenen Titandioxid-Nanopartikeln ausgebildet. Durch die Verwendung von Titandioxid-Nanopartikeln, welche bei Sonnenschutz- und Pflegeprodukten Anwendung finden, kann die wirksame Oberfläche, welche zur Keimentwicklung hemmenden Wirkung beiträgt, vergrößert werden. Die Verwendung von Titandioxid-Nanopartikeln ist vorteilhaft, da deren Größe in einem Bereich von 0,2 Nanometer bis 100 Nanometer gut einstellbar ist. Damit kann mittels der Einstellung der Größe der Titandioxid-Nanopartikel die wirksame, zur Hemmung der Keimentwicklung zur Verfügung stehende Oberfläche eingestellt werden.

Zum anderen sind für Titandioxid trotz jahrelangem Einsatz in Sonnenschutz- und Pflegeprodukten keine belastenden Nebenwirkungen für menschliche Patienten bekannt. Weiterhin weisen in der Regel Silber-beschichtete Titandioxid-Nanopartikel niedrigere Herstellungskosten auf als reine Silber-Nanopartikel, da weniger teures Silber benötigt wird. Alternativ zu Titandioxid Nanopartikeln können auch Nanopartikel anderer Zusammensetzung, wie bspw. Zinkoxid-Nanopartikel, verwendet werden. Auf eine Silberbeschichtung von Nanopartikeln kann verzichtet werden, wenn die Nanopartikel an sich und/oder als Schicht eine Keimentwicklung-hemmende Wirkung aufweisen.

Gegenüber der Silberbeschichtung zur Lösung der Aufgabe, wobei die Silberschicht bspw. durch Aufdampfen bereitgestellt werden kann, weisen Silber-beschichtete Nanopartikel den Vorteil auf, dass die Eigenabsorption der Röntgenstrahlen gegenüber massiven Silberschichten oder massiven Silber-Nanopartikeln reduziert ist.

In einer weiteren vorteilhaften Ausgestaltung der Erfindung ist die Beschichtung als wenigstens eine Lage von C60-Aggregaten ausgebildet. Dabei werden Kumulationen von C60-Fullerenen, so genannte Aggregate, als in einer Lösung vorliegende Kolloide auf wenigstens einen Teil des Objekttisches und/oder auf wenigstens einen Teil der Kompressionsplatte aufgebracht. Die Kolloid-Aggregate aus C60-Molekülen weisen dabei eine Keimentwicklung-hemmende Wirkung auf sowie ein gegenüber einem Untersuchungsobjekt inertes Verhalten und eignen sich daher für die Anwendung für eine erfindungsgemäße Röntgenvorrichtung. Auch durch den Einsatz von C60-Kolloid-Aggregaten wird die für die Hemmung der Keimentwicklung zur Verfügung wirksame Oberfläche erhöht.

Eine Verwendung von C60-Aggregaten als Beschichtung zur Hemmung der Keimentwicklung ist insbesondere deshalb vorteilhaft, da die C60-Moleküle aus 60 Kohlenstoffatomen aufgebaut sind, wobei das Element Kohlenstoff eine geringe Kernladungszahl aufweist. Die geringe Kernladungszahl von Kohlenstoff führt auch bei höheren Schichtdicken nur zu einer geringen Absorption von Röntgenstrahlen, was für die vorliegende Erfindung einen Vorteil darstellt.

In einer vorteilhaften Ausführungsvariante der Erfindung ist in wenigstens einem Teil des Objekttisches und/oder in wenigstens einem Teil der Kompressionsplatte wenigstens ein die Keimentwicklung hemmendes Element und/oder wenigstens ein die Keimentwicklung hemmender Stoff eingelagert. Es werden also während der Herstellung einer Kompressionsplatte und/oder eines Objekttisches, welche bspw. aus Polymethylmethacrylat (PMMA) gefertigt wird, ein Element und/oder ein Stoff mit Keimentwicklung-hemmender Wirkung eingearbeitet bzw. in das Material, hier PMMA, eingelagert. Es bedarf als keiner zusätzlichen Beschichtung für einen fertig gestellten Objekttisch und/oder eine fertig gestellte Kompressionsplatte, um eine Keimentwicklung-hemmende Wirkung bereitzustellen.

Vorteilhaft können Silber-Ionen zur Bereitstellung einer Keimentwicklung-hemmenden Wirkung bspw. in PMMA eingebettet werden. Die Silber-Ionen befinden sich durch die Einbettung in das PMMA auch an der jeweiligen Grenzfläche, an welcher das zu untersuchende Teil des Untersuchungsobjekts - etwa eine weibliche Brust - mit dem Objekttisch bzw. der Kompressionsplatte in Kontakt kommt. Der Kontakt von Keimen mit den Silber-Ionen an der Grenzfläche des Objekttisches bzw. der Kompressionsplatte führt zu einer Hemmung der Keimentwicklung in Form von Keimabtötung.

Weitere Vorteile der Erfindung ergeben sich aus einem schematischen Ausführungsbeispiel, welches anhand der nachfolgenden Zeichnungen genauer erläutert wird. Es zeigen:
- FIG 1: eine Seitenansicht einer Mammographievorrichtung mit einer erfindungsgemäßen die Keimentwicklung hemmenden Beschichtung eines Objekttisches und einer Kompressionsplatte,
- FIG 2: eine Draufsicht auf einen mit einer die Keimentwicklung hemmenden Beschichtung beschichteten Objekttisch,
- FIG 3: eine Seitenansicht einer Mammographievorrichtung mit die Keimentwicklung-hemmenden Silber-Ionen, eingebettet in das PMMA eines Objekttisches und einer Kompressionsplatte,
- FIG 4: eine Draufsicht auf einen mit eingebetteten Silber-Ionen versehenen Objekttisch.

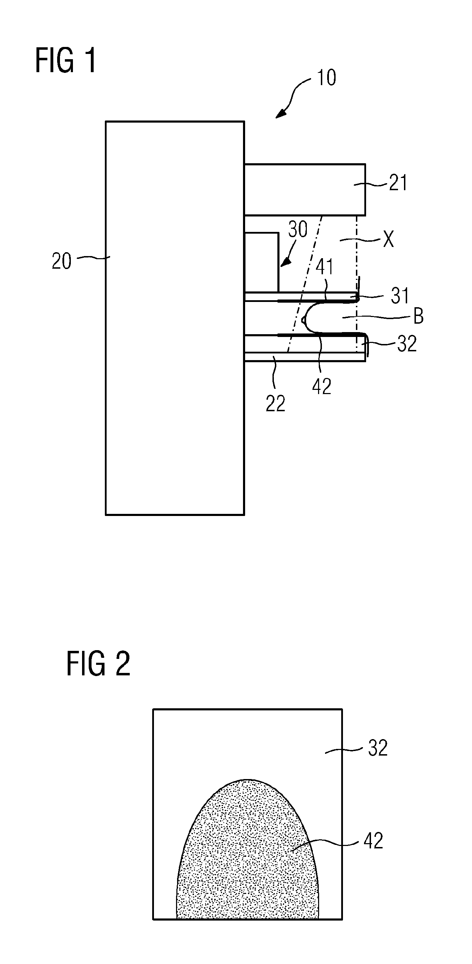

FIG 1 zeigt eine Mammographievorrichtung 10 zur Durchführung von Röntgenuntersuchungen, insbesondere an einer weiblichen Brust B eines menschlichen Patienten. Die Mammographievorrichtung 10 weist eine Stativeinheit 20 sowie einen Röntgenstrahler 21 und einen Röntgendetektor 22 auf. Der Röntgendetektor 22 ist unterhalb einer Oberseite eines Objekttisches 32 angeordnet, so dass bei einer Röntgenuntersuchung die weibliche Brust B zwischen Röntgenstrahler 21 und Röntgendetektor 22 in einem Strahlbereich X auf dem Objekttisch 32 positionierbar ist. Weiter weist die Mammographievorrichtung 10 eine Kompressionseinrichtung 30 mit einer Kompressionsplatte 31 auf. Die Kompressionseinrichtung 30 ist relativ zum Objekttisch vertikal verstellbar. Dadurch kann der Abstand der Kompressionsplatte 31 zum Objekttisch 32 justiert werden.

Mittels der Kompressionsplatte 31 und des Objekttisches 32 kann die weibliche Brust B vor der Strahlenexposition komprimiert werden. Durch die Kompression des zu untersuchenden Teils des Untersuchungsobjekts, hier der weiblichen Brust B, werden Röntgenstreustrahlen reduziert und eine im Wesentlichen konstante Dicke für die Durchstrahlung der weiblichen Brust B mittels Röntgenstrahlen bereitgestellt. Im komprimierten Zustand der weiblichen Brust B steht die weibliche Brust B in direktem Kontakt mit der Unterseite der Kompressionsplatte 31 und der Oberseite des Objekttisches 32.

Während der Kompression der Brust B werden in der Regel von der zu untersuchenden Person bzw. der zu untersuchenden weiblichen Brust B Keime auf die Kompressionsplatte 31 und den Objekttisch 32 übertragen. Da die Kompressionsplatte 31 zwischen einer Untersuchung an einem ersten Patienten und einer Untersuchung an einem zweiten Patienten in der Regel nicht gewechselt wird, wenn ähnliche anatomische Gegebenheiten der Patienten und ähnliche medizinische Fragestellungen gegeben sind, kann eine Übertragung von Keimen von dem ersten Patienten zu dem zweiten Patienten erfolgen.

Um eine Übertragung von Keimen von dem ersten Patienten auf den zweiten Patienten zu verhindern, weisen sowohl der Objekttisch 32 als auch die Kompressionsplatte 31 einen Teil 42 bzw. 41 auf, welcher eine Keimentwicklung-hemmende Wirkung besitzt. Die Keimentwicklung hemmende Wirkung besteht im Ausführungsbeispiel in einer Keimabtötung. Die keimabtötende Wirkung wird durch eine Beschichtung des Teils 41 der Kompressionsplatte 31 und des Teils 42 des Objekttisches 32 erreicht, wobei die Beschichtung als eine Lage aus einer Vielzahl von Silber-beschichteten Titanoxid-Nanopartikel erreicht wird.

Der Teil 42 des Objekttisches 32 mit keimabtötender Wirkung und der Teil 41 der Kompressionsplatte 31 mit keimabtötender Wirkung sind derart ausgebildet, dass diese einer komprimierten Brust B in Form und Größe im Wesentlichen angepasst sind. D.h. eine komprimierte Brust berührt im komprimierten Zustand nur einen bestimmten Bereich des Objekttisches 32 bzw. nur einen bestimmten Bereich der Kompressionsplatte 31, welche eine keimabtötende Beschichtung aufweisen, wobei dieser Bereich nicht über den Teil 41 bzw. 42 mit der die Keimentwicklung hemmenden Beschichtung hinausragt. Dadurch kann eine Übertragung von Keimen eines ersten Patienten auf einen zweiten Patienten unterbunden werden.

Die von dem ersten Patienten auf die Kompressionsplatte 31 und/oder auf den Objekttisch 32 übertragenen Keime werden durch das Silber, welches die Titandioxid-Nanopartikel umgibt, abgetötet. Dadurch werden keine lebenden Mikroorganismen auf nachfolgende Patienten übertragen und die Infektionsgefahr durch lebende Mikroorganismen wird deutlich verringert.

FIG 2 zeigt einen Ausschnitt eines Objekttisches 32, auf welchem eine erfindungsgemäße Beschichtung zur Hemmung einer Keimentwicklung vorgesehen ist. Der beschichtete Teil 42 des Objekttisches 32 ist im vorliegenden Fall halbelliptisch ausgebildet. Der beschichtete Teil kann jedoch auch beliebig in anderer Weise geformt sein. Es kann auch vorgesehen werden, den gesamten Objekttisch 32 oder auch nur die Oberseite des Objekttisches 32 mit einer die Keimentwicklung hemmenden Beschichtung 42 zu versehen. Analog gilt dies für die erfindungsgemäße Beschichtung der Kompressionsplatte 31.

Zur Vermeidung einer höheren Röntgendosis für den Patienten, welche für einen Objekttisch 32 mit Silberbeschichtung oder für einen Objekttisch 32 mit in das PMMA inkorporierte Silber-Ionen auftreten kann, kann die Keimentwicklung hemmende Wirkung durch C60-Aggregate erreicht werden.

Bei Nutzung einer Silber-haltigen Beschichtung zur Hemmung der Keimentwicklung für die Kompressionsplatte 31 kann zwar die eingestrahlte absolute Röntgendosis erhöht sein, jedoch wird ein Teil durch die keimabtötende Silberbeschichtung der Kompressionsplatte 31, noch bevor die Röntgenstrahlen in die weibliche Brust B des Untersuchungsobjekts eintreten, absorbiert. D.h. die Röntgendosis für das Untersuchungsobjekt wird nicht erhöht. Eine Erhöhung der Röntgendosis für das Untersuchungsobjekt ist nur dann erforderlich, wenn nach Durchdringen des Untersuchungsobjekts bzw. der weiblichen Brust B eine weitere Absorption durch eine Silber-haltige keimabtötende Schicht erfolgt, bevor die Röntgenstrahlen auf den Röntgendetektor 22 treffen. Die Verwendung von C60-Aggregaten ist somit als desinfizierende bzw. antibakterielle Beschichtung des Objekttisches 32 im Hinblick auf die Röntgendosis für den Patienten vorteilhaft.

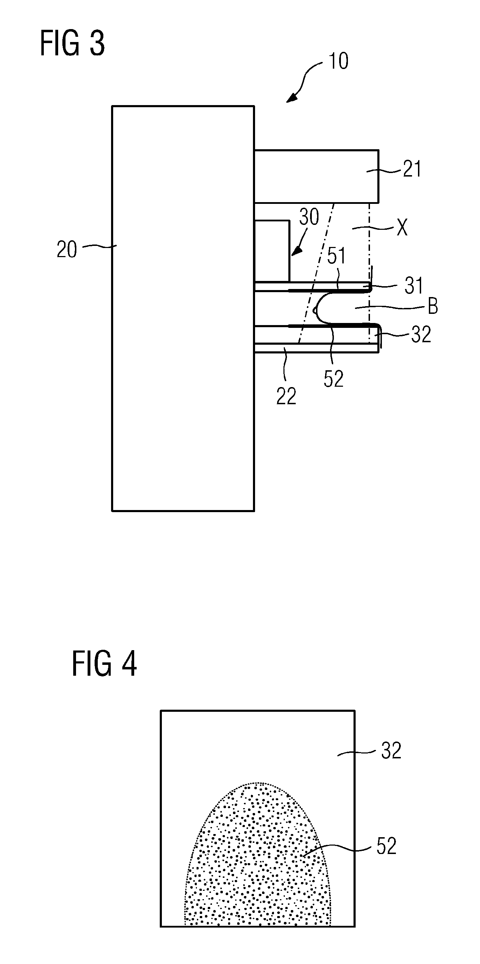

FIG 3 zeigt eine Seitenansicht einer Mammographievorrichtung 10 zur Durchführung einer Röntgenuntersuchung insbesondere an einer weiblichen Brust B. Die Mammographievorrichtung 10 weist wie in FIG 1 eine Stativeinheit 20 auf, sowie einen Röntgenstrahler 21 und einen Röntgendetektor 22. Der Röntgendetektor 22 ist unterhalb einer Oberseite eines Objekttisches 32 angeordnet, so dass bei einer Röntgenuntersuchung die weibliche Brust B zwischen Röntgenstrahler 21 und Röntgendetektor 22 in einem Strahlbereich X auf dem Objekttisch 32 positionierbar ist. Weiter weist die Mammographievorrichtung 10 eine Kompressionseinrichtung 30 mit einer Kompressionsplatte 31 auf. Die Kompressionseinrichtung 30 ist relativ zum Objekttisch 32 vertikal verstellbar. Dadurch kann der Abstand der Kompressionsplatte 31 zum Objekttisch 32 justiert und der Kompressionsdruck auf die weibliche Brust B eingestellt werden.

Mittels der Kompressionsplatte 31 und des Objekttisches 32 kann die weibliche Brust B vor der Strahlenexposition komprimiert werden. Durch die Kompression der Brust B werden Röntgenstreustrahlen reduziert und eine im Wesentlichen konstante Dicke für die Durchstrahlung der weiblichen Brust B mittels Röntgenstrahlen bereitgestellt. Im komprimierten Zustand der weiblichen Brust B steht diese in direktem Kontakt mit der Unterseite der Kompressionsplatte 31 und der Oberseite des Objekttisches 32.

Während der Kompression der Brust B werden in der Regel von der zu untersuchenden Person bzw. der zu untersuchenden weiblichen Brust B Keime auf die Kompressionsplatte 31 und den Objekttisch 32 übertragen. Da die Kompressionsplatte 31 zwischen einer Untersuchung an einem ersten Patienten und einer Untersuchung an einem zweiten Patienten in der Regel nicht gewechselt wird, wenn ähnliche anatomische Gegebenheiten der Patienten und ähnliche medizinische Fragestellungen gegeben sind, kann eine Übertragung von Keimen von dem ersten Patienten auf den zweiten Patienten erfolgen.

Um eine Übertragung von einem ersten Patienten auf einen zweiten Patienten zu vermeiden, sind in FIG 3 in einem bestimmten Teil 51 bzw. 52 der Kompressionsplatte 31 bzw. des Objekttisches 32 in die Kompressionsplatte 31 und in den Objekttisch 32 eingelagerte Silber-Ionen vorgesehen. Die Silber-Ionen werden während der Herstellung des bspw. aus Polymethylmethacrylat bestehenden Objekttisches 32 und der bspw. aus Polymethylmethacrylat bestehenden Kompressionsplatte 31 in einen vorgesehenen Teil 51 bzw. 52 eingebracht. Vorzugsweise werden die Silber-Ionen möglichst oberflächennah positioniert, damit eine möglichst große Wirkung gegenüber Keimen erreicht wird. Dabei ist darauf zu achten, dass die Flächenkonzentration von Silber-Ionen derart bemessen ist, dass es zu keiner oder nur zu einer vernachlässigbaren Beeinflussung der Röntgenstrahlen infolge von Absorption durch die Silber-Ionen erfolgt.

FIG 4 zeigt eine Draufsicht eines Objekttisches 32, welcher im Wesentlichen aus Polymethylmethacrylat (PMMA) gefertigt ist. Während der Fertigung des Objekttisches 32 werden Silber-Ionen, vorzugsweise oberflächennah, in einen bestimmten Teil 52 des Objekttisches 32 eingelagert. Die eingelagerten Silber-Ionen besitzen bei Kontakt mit Keimen eine die Keimentwicklung hemmende Wirkung, indem sie diese abtöten. Vorzugsweise ist nicht der gesamte Objekttisch 32 mit Silber-Ionen durchsetzt, sondern nur jener Teil 52 des Objekttisches 32, welcher mit dem zu untersuchenden Teil des Untersuchungsobjekts, im vorliegenden Fall die weibliche Brust B, physisch direkt in Kontakt tritt. Der Teil 52 des Objekttisches 32 ist in FIG 4 elliptisch ausgebildet. Jedoch ist auch eine beliebige andere Form ausführbar. Analoge Betrachtungen gelten für die aus FIG 3 bekannte Kompressionsplatte 31 und für ein von der Kompressionsplatte 31 umfassten Teil 51, welcher zur Hemmung der Keimentwicklung vorgesehen ist.

Die Einlagerung von Silber-Ionen oder anderer Elemente mit Keimentwicklung-hemmender Wirkung bzw. anderen Stoffen mit Keimentwicklung-hemmender Wirkung kann auch nach Fertigstellung des Objekttisches 32 erfolgen. Denkbar sind bspw. Implantationsprozesse oder Diffusionsprozesse für die entsprechenden Elemente und/oder Stoffe, um diese in das Material des Objekttisches 32 einzubringen. Anstatt von Polymethylmethacrylat für die Fertigung einer Kompressionsplatte 31 oder eines Objekttisches 32 können auch andere Materialen mit guter Röntgentransparenz und sonstigen geeigneten Eigenschaften - z.B. mechanische, chemische - eingesetzt werden.

## Patentansprüche

1. Röntgenvorrichtung (10) zur Bildaufnahme eines zu untersuchenden Teils (B) eines Untersuchungsobjekts, mit einem Röntgenstrahler (21) und einem Röntgendetektor (22), zwischen welchen sich ein Strahlbereich (X) erstreckt, in dem der zu untersuchende Teil (B) des Untersuchungsobjekts positionierbar ist, wobei ein Objekttisch (32) zur Positionierung des zu untersuchenden Teils (B) des Untersuchungsobjekts im Strahlbereich (X) und eine relativ zum Objekttisch (32) verstellbare Kompressionseinrichtung (30) mit einer Kompressionsplatte (31) vorgesehen sind, wobei der zu untersuchende Teil (B) des Untersuchungsobjekts mittels des Objekttisches (32) und der Kompressionsplatte (31) komprimierbar ist,
**dadurch gekennzeichnet, dass** wenigstens ein Teil (41, 51) der Kompressionsplatte (31) und/oder wenigstens ein Teil (42, 52) des Objekttisches (32) derart ausgebildet ist, dass eine Hemmung einer Keimentwicklung bewirkt wird.

2. Röntgenvorrichtung nach Anspruch 1,
**dadurch gekennzeichnet, dass**
wenigstens ein Teil (41, 51) der Kompressionsplatte (31) und/oder wenigstens ein Teil (42, 52) des Objekttisches (32) eine Keimentwicklung hemmende Beschichtung (41, 42) aufweist.

3. Röntgenvorrichtung nach Anspruch 2,
**dadurch gekennzeichnet, dass**
die Beschichtung als eine Silberschicht ausgebildet ist.

4. Röntgenvorrichtung nach Anspruch 2,
**dadurch gekennzeichnet, dass** die Beschichtung (41, 42) als eine Vielzahl von jeweils mit Silber überzogenen Titandioxid-Nanopartikeln ausgebildet ist.

5. Röntgenvorrichtung nach Anspruch 2,
**dadurch gekennzeichnet, dass** die Beschichtung als wenigstens eine Lage von C60-Aggregaten ausgebildet ist.

6. Röntgenvorrichtung nach Anspruch 1,
**dadurch gekennzeichnet, dass** in wenigstens einem Teil (42, 52) des Objekttisches (32) und/oder in wenigstens einem Teil (41, 51) der Kompressionsplatte (31) wenigstens ein die Keimentwicklung hemmendes Element und/oder wenigstens ein die Keimentwicklung hemmender Stoff eingelagert ist.

7. Röntgenvorrichtung nach Anspruch 6,
**dadurch gekennzeichnet, dass** das die Keimentwicklung hemmende Element Silber ist.
